(19) Europäisches Patentamt
European Patent Office
Office européen des brevets

(11) EP 1 312 352 B1

# (12) FASCICULE DE BREVET EUROPEEN

(45) Date de publication et mention de la délivrance du brevet:
**12.01.2011 Bulletin 2011/02**

(51) Int Cl.:
***A61K 8/898*** *(2006.01)* ***A61Q 5/10*** *(2006.01)*
***A61Q 5/04*** *(2006.01)*

(21) Numéro de dépôt: **02292747.9**

(22) Date de dépôt: **04.11.2002**

(54) **Composition oxydante pour le traitement des fibres kératiniques comprenant une silicone aminée particulière**

Oxidative Zusammensetzung zur Behandlung von Keratinfasern, die spezielle Aminosilikone enthält

Oxidising composition for keratin fibers containing aminosilicone

(84) Etats contractants désignés:
**AT BE BG CH CY CZ DE DK EE ES FI FR GB GR IE IT LI LU MC NL PT SE SK TR**

(30) Priorité: **08.11.2001 FR 0114474**

(43) Date de publication de la demande:
**21.05.2003 Bulletin 2003/21**

(73) Titulaire: **L'Oréal**
**75008 Paris (FR)**

(72) Inventeurs:
- **Legrand, Frédéric**
  **92400 Courbevoie (FR)**
- **Millequant, Jean-Marie**
  **94100 Saint-Maur des Fosses (FR)**

(74) Mandataire: **Fevrier, Murielle Françoise E.**
**L'Oréal**
**D.I.P.I.**
**25-29 Quai Aulagnier**
**92600 Asnières-sur-Seine Cedex (FR)**

(56) Documents cités:
**EP-A- 0 890 355** **DE-A- 19 754 053**
**GB-A- 2 141 454** **US-A- 5 340 367**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 312 352 B1

## Description

La présente invention a trait à une composition oxydante, destinée au traitement des fibres kératiniques humaines et plus particulièrement des cheveux comprenant une silicone aminée particulière, ainsi que ses utilisations pour la teinture, pour la déformation permanente ou pour la décoloration desdites fibres.

Il est connu de décolorer les fibres kératiniques et en particulier les cheveux humains, avec des compositions de décoloration contenant un ou plusieurs agents oxydants. Parmi les agents oxydants classiquement utilisés, on peut citer le peroxyde d'hydrogène ou des composés susceptibles de produire le peroxyde d'hydrogène par hydrolyse, tels que le peroxyde d'urée ou les persels comme les perborates, les percarbonates et les persulfates, le peroxyde d'hydrogène et les persulfates étant particulièrement préférés.

Lesdites compositions de décoloration se présentent principalement sous forme de produits anhydres ( poudres ou crèmes ) contenant des composés alcalins (amines et silicates alcalins), et un réactif peroxygéné tels que les persulfates, perborates ou percarbonates, d'ammonium ou de métaux alcalins, que l'on dilue au moment de l'emploi avec une composition aqueuse de peroxyde d'hydrogène.

Les compositions de décoloration peuvent aussi résulter du mélange, au moment de l'emploi, de la poudre anhydre de réactif péroxygéné avec une composition aqueuse contenant les composés alcalins et une autre composition aqueuse contenant le peroxyde d'hydrogène.

Les compositions de décoloration se présentent également sous forme de compositions aqueuses épaissies de peroxyde d'hydrogène, prêtes à l'emploi.

Par "composition prête à l'emploi" on entend, au sens de l'invention, la composition destinée à être appliquée telle quelle sur les fibres kératiniques, c'est à dire qu'elle peut être stockée telle quelle avant utilisation ou résulter du mélange extemporané de deux ou plusieurs compositions.

Il est connu par ailleurs de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants. On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène ou des composés susceptibles de produire le peroxyde d'hydrogène par hydrolyse tels que le peroxyde d'urée, les persels comme les perborates et persulfates. Le peroxyde d'hydrogène est particulièrement préféré.

On sait également que la technique la plus usuelle pour obtenir une déformation permanente des cheveux consiste, dans un premier temps, à réaliser l'ouverture des liaisons disulfures -S-S- de la kératine (cystine) à l'aide d'une composition contenant un agent réducteur adapté (étape de réduction) puis, après avoir rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation temporaire, telles que de mise en pli.

Les compositions réductrices utilisables pour la mise en oeuvre de la première étape d'une opération de permanente contiennent généralement, à titre d'agents réducteurs, des sulfites, des bisulfites, des alkyl-phosphines ou de préférence des thiols.

Les compositions oxydantes nécessaires à la mise en oeuvre de l'étape de fixation, font le plus souvent appel, dans la pratique, à des compositions à base d'eau oxygénée.

Par ailleurs, il est connu que les traitements oxydants de la déformation permanente des cheveux et surtout ceux de la coloration et de la décoloration sont souvent agressifs et conduisent à de mauvaises propriétés cosmétiques des cheveux tels qu'un démêlage difficile, un toucher désagréable, ou des cheveux rêches, ternes ou encore chargés d'électricité statique, et à une dégradation des fibres.

EP890335 décrit une composition cosmétique pour les cheveux comprenant un agent oxydant et une silicone aminée. La demanderesse, après d'importantes recherches menées sur la question, vient maintenant de découvrir qu'en utilisant une silicone aminée de formules (I) ou (II) définies ci-après dans une composition oxydante pour la

déformation permanente, la coloration et la décoloration des fibres kératiniques humaines, elle pouvait remédier à tous ces inconvénients, avec des effets conditionneurs et rémanents supérieurs à ceux des systèmes antérieurement utilisés, sans pour autant altérer la puissance et l'homogénéité desdites déformations permanentes, colorations et décolorations.

Ainsi l'état des fibres est amélioré et celles-ci conservent leur toucher doux, leur facilité de démêlage et leur brillance après plusieurs shampooings.

Par amélioration de l'état de la fibre on entend une diminution de la porosité ou de la solubilité alcaline de la fibre et une amélioration des propriétés cosmétiques et en particulier du lissage, de la douceur et de la facilité de démêlage et de coiffage.

Cet effet est rémanent, c'est à dire durable.

La porosité se mesure par la fixation à 37°C et à pH10, en 2 minutes, de la 2-nitro paraphénylènediamine à 0,25% dans un mélange éthanol / tampon pH 10 (rapport volumique 10/90).

La solubilité alcaline correspond à la perte de masse d'un échantillon de 100 mg de fibres kératiniques sous l'action de la soude décinormale pendant 30 minutes à 65°C.

La présente invention a donc pour objet une composition cosmétique destinée au traitement des fibres kératiniques humaines et plus particulièrement des cheveux, comprenant dans un milieu cosmétiquement acceptable:

(i) au moins un agent oxydant et,
(ii) au moins une silicone aminée de formules (I) ou (II) décrites ci-après.

Mais d'autres caractéristiques, aspects, objets et avantages de l'invention apparaîtront encore plus clairement à la lecture de la description et des exemples qui suivent.

<u>Silicones aminées</u>

Les silicones aminées de formules (I) ou (II) selon l'invention sont les suivantes :

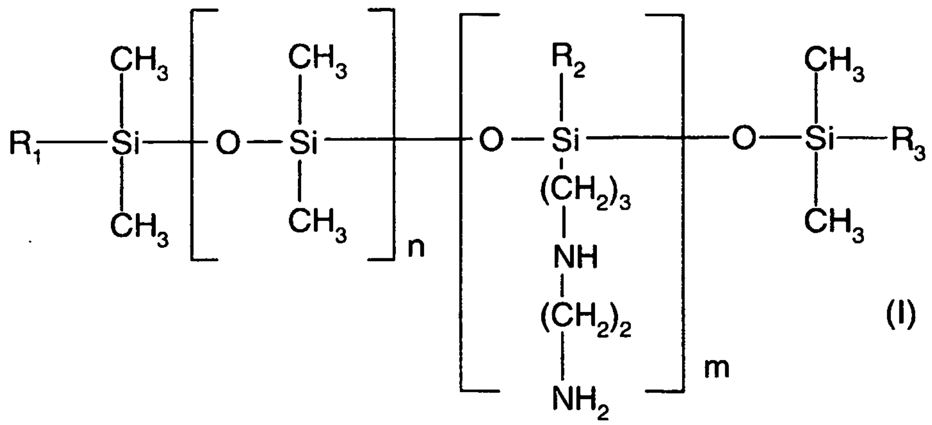

(I)

dans laquelle :

m et n sont des nombres tels que la somme (n + m) peut varier notamment de 1 à 1 000 et en particulier de 50 à 250 et plus particulièrement de 100 à 200,
n pouvant désigner un nombre de 0 à 999 et notamment de 49 à 249 et plus particulièrement de 125 à 175 et m pouvant désigner un nombre de 1 à 1 000, et notamment de 1 à 10 et plus particulièrement de 1 à 5;
$R_1$, $R_2$, $R_3$, identiques ou différents, représentent un radical hydroxy ou alcoxy en $C_1$-$C_4$, l'un au moins des radicaux $R_1$ à $R_3$ désignant un radical alcoxy.

De préférence le radical alcoxy est un radical méthoxy.

Le rapport molaire Hydroxy/Alcoxy est de préférence compris entre 0,2:1 et 0,4:1 et de préférence entre 0,25:1 et 0,35:1 et plus particulièrement est égal à 0,3.

La silicone aminée de formule (I) présente une masse moléculaire moyenne en poids allant de préférence de 2000 à 1000000 et encore plus particulièrement de 3500 à 200000.

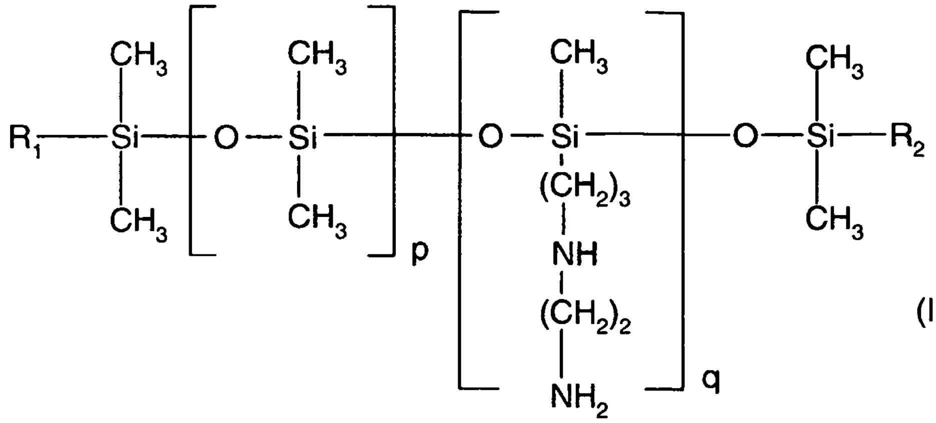

(II)

dans laquelle :

p et q sont des nombres tels que la somme (p + q) peut varier notamment de 1 à 1 000 et en particulier de 50 à 350, et plus particulièrement de 150 à 250
p pouvant désigner un nombre de 0 à 999 et notamment de 49 à 349 et plus particulièrement de 159 à 239 et q pouvant désigner un nombre de 1 à 1 000, et notamment de 1 à 10 et plus particulièrement de 1 à 5;
$R_1$, $R_2$, différents, représentent un radical hydroxy ou alcoxy en $C_1$-$C_4$, l'un au moins des radicaux $R_1$ à $R_2$ désignant un radical alcoxy.

De préférence le radical alcoxy est un radical méthoxy.

Le rapport molaire Hydroxy/Alcoxy est de préférence compris entre 1:0,8 et 1:1,1 et de préférence entre 1:0,9 et 1:1 et plus particulièrement est égal à 1:0,95.

La silicone aminée de formule (II) présente une masse moléculaire moyenne en poids allant de préférence de 2000 à 200.000, et encore plus particulièrement de 5.000 à 100.000 et plus particulièrement encore de 10.000 à 50.000.

Les masses moléculaires moyennes en poids de ces silicones aminées sont mesurées par Chromatographie par Perméation de Gel (GPC) à température ambiante en équivalent polystyrène. Les colonnes utilisées sont des colonnes $\mu$ styragel. L'éluant est le THF, le débit est de 1 ml/mn. On injecte 200 $\mu$l d'une solution à 0,5% en poids de silicone dans le THF. La détection se fait par réfractométrie et UVmétrie.

Les produits commerciaux correspondant à ces silicones de structure (I) ou (II) peuvent inclure dans leur composition une ou plusieurs autres silicones aminées dont la structure est différente des structures (I) et (II).

Un produit contenant des silicones aminées de structure (I) est proposé par la société WACKER sous la dénomination BELSIL ADM 652®.

Des produits contenant des silicones aminées de structure (II) sont proposés par la société WACKER sous la dénomination Fluid WR 1300®, et BELSIL ADM 6057®.

Lorsque ces silicones aminées sont mises en oeuvre, une forme de réalisation particulièrement intéressante est leur utilisation sous forme d'émulsion huile-dans-eau. L'émulsion huile-dans-eau peut comprendre un ou plusieurs tensioactifs. Les tensioactifs peuvent être de toute nature mais de préférence cationique et/ou non ionique.

Les particules de silicone dans l'émulsion ont une taille moyenne allant généralement de 3 nm à 500 nanomètres.

De préférence, notamment pour les silicones aminées de formule (II), on utilise des microémulsions de taille allant de 5 nm à 60 nanomètres et plus particulièrement de 10 nm à 50 nanomètres.

On peut utiliser selon l'invention les microémulsions de silicones aminées de formule (II) proposées sous la dénomination FINISH CT 96 E® ou SLM 28020® par la société WACKER.

De préférence la silicone aminée de formule (I) ou (II) est choisie de telle façon que l'angle de contact avec l'eau d'un cheveu traité avec une composition contenant 2% MA (matières actives) de ladite silicone selon l'invention soit compris entre 90 et 180° et de préférence entre 90 et 130°, bornes incluses.

De préférence la composition contenant la ou les silicones aminées de formule (I) ou (II) est telle que l'angle de contact d'un cheveu traité avec la dite composition est compris entre 90 et 180° et de préférence entre 90 et 130°, bornes incluses.

La mesure de l'angle de contact est basée sur l'immersion d'un cheveu dans de l'eau distillée. Il consiste à évaluer la force exercée par l'eau sur le cheveu lors de son immersion de l'eau distillée et lors de son retrait. Les forces ainsi mesurées sont directement reliées à l'angle de contact θ entre l'eau et la surface du cheveu. Le cheveu est dit hydrophile lorsque l'angle θ est compris entre 0 et 90°, hydrophobe lorsque cet angle est compris entre 90° et 180°,

bornes incluses.

Le test s'effectue avec des mèches de cheveux naturels ayant été décolorés dans les mêmes conditions puis lavés.

Chaque mèche de 1 g est placée dans un cristallisoir de 75 mm de diamètre puis recouverte de façon homogène par 5 mL de la formule à tester. La mèche est ainsi laissée 15 minutes à température ambiante puis rincée pendant 30 secondes. La mèche essorée est laissée à l'air libre jusqu'à ce qu'elle soit complètement sèche.

Pour chaque évaluation, 10 cheveux ayant subi le même traitement sont analysés. Chaque échantillon, fixé à une microbalance de précision, est immergé par la pointe dans un récipient rempli d'eau distillée. Cette balance DCA (« Dynamic Contact Angle Analyser »), de la société CAHN Instruments, permet de mesurer la force (F) exercée par l'eau sur le cheveu.

Parallèlement, le périmètre du cheveu (P) est mesuré *via* une observation microscopique. La force moyenne de mouillabilité sur 10 cheveux et la section des cheveux analysés permettent d'obtenir l'angle de contact du cheveu sur l'eau, selon la formule :

$$F = P * \Gamma lv * \cos\theta$$

où F est la force de mouillabilité exprimée en Newton, P le périmètre du cheveu en mètre, $\Gamma lv$ la tension interfaciale liquide / vapeur de l'eau en $J/m^2$ et $\theta$ l'angle de contact.

Le produit SLM 28020® de WACKER à 12% dans l'eau (soit 2% en silicone aminée) conduit à un angle de contact de 93° selon le test indiqué ci-dessus.

La silicone aminée de formule (I) ou (II) est utilisée de préférence dans la composition oxydante selon l'invention en une quantité allant de 0,01 à 20% en poids du poids total de la composition. Plus préférentiellement, cette quantité va de 0,1 à 15% en poids et encore plus particulièrement de 0,5 à 10 % en poids.

<u>Agent oxydant</u>

Selon l'invention l'agent oxydant est de préférence choisi dans le groupe constitué par le peroxyde d'hydrogène et les composés susceptibles de produire par hydrolyse du peroxyde d'hydrogène, ou leurs mélanges.

L'agent oxydant de la composition selon l'invention est choisi de préférence dans le groupe formé par l'eau oxygénée, le peroxyde d'urée, les persels tels que les perborates ou les persulfates ou leurs mélanges.

Plus particulièrement, l'agent oxydant est le peroxyde d'hydrogène, et plus préférentiellement encore, l'agent oxydant est de l'eau oxygénée.

La concentration en peroxyde d'hydrogène peut varier de 0,5 à 40 volumes, de préférence de 2 à 30 volumes et celle en composé susceptible de former du peroxyde d'hydrogène par hydrolyse de 0,1 à 25 % en poids par rapport au poids total de la composition oxydante.

Les compositions oxydantes selon l'invention peuvent être anhydres ou aqueuses.

Les compositions oxydantes selon l'invention sont de préférence aqueuses et le pH de l'ensemble d'une composition oxydante aqueuse varie de préférence de 1 à 13, et encore plus préférentiellement de 2 à 12.

La composition oxydante peut également se présenter, en particulier dans le cas de la décoloration, sous forme de deux parties à mélanger au moment de l'emploi, l'une de ces deux parties contenant des agents alcalins et se présentant sous forme solide ou liquide. Pour le peroxyde d'hydrogène, le pH est de préférence inférieur à 7 avant mélange.

Le pH des compositions aqueuses oxydantes selon l'invention peut être obtenu et/ou ajusté classiquement par ajout soit d'agents basifiants, tels que par exemple l'ammoniaque, la monoéthanolamine, la diéthanolamine, la triéthanolamine, l'isopropanolamine, la propanediamine-1,3, un carbonate ou bicarbonate alcalin ou d'ammonium, un carbonate organique tel que le carbonate de guanidine, ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique ou l'acide borique.

La composition oxydante peut contenir des additifs connus pour leur utilisation dans les compositions oxydantes pour la teinture des cheveux par oxydation, pour la déformation permanente ou la décoloration des cheveux tels que des agents alcalinisants ou acidifiants, des agents conservateurs, ou des agents séquestrants.

De préférence, lorsque l'agent oxydant est de l'eau oxygénée, la composition oxydante selon l'invention contient au moins un agent stabilisant de l'eau oxygénée. Dans les compositions associant l'eau oxygénée et les polymères amphiphiles de la présente invention, des résultats particulièrement avantageux ont été obtenus en utilisant au moins un agent stabilisant choisi parmi les pyrophosphates des métaux alcalins ou alcalino-terreux, les stannates des métaux alcalins ou alcalino-terreux, la phénacétine ou les sels d'acides et d'oxyquinoléine, comme le sulfate d'oxyquinoléine.

De manière plus avantageuse encore, on utilise au moins un stannate en association ou non à au moins un pyrophosphate.

Dans les compositions oxydantes selon l'invention, la concentration en agents stabilisants de l'eau oxygénée peut varier de 0,0001 % à 5% en poids et de préférence de 0,01 à 2% en poids par rapport au poids total des compositions oxydantes.

Dans les compositions oxydantes selon l'invention à l'eau oxygénée, le rapport des concentrations du peroxyde d'hydrogène aux agents stabilisants peut varier de 0,05 à 1000 et de préférence de 0,1 à 500 et plus préférentiellement encore de 1 à 200. De préférence, selon l'invention, le rapport des concentrations de la silicone aminée selon l'invention aux agents oxydants est compris entre 0,001 et 10, les quantités desdits silicones et oxydants étant exprimées en matières actives (peroxyde d'hydrogène pour l'eau oxygénée). Plus préférentiellement encore, ce rapport est compris entre 0,01 et 5 et plus particulièrement encore entre 0,02 et 1.

Milieu

Le milieu de la composition cosmétiquement acceptable, est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,5 et 20% et, de préférence, entre environ 2 et 10% en poids par rapport au poids total de la composition.

Agents ingrédients

Les compositions selon l'invention peuvent également comprendre des agents d'ajustement de la rhéologie tels que les épaississants cellulosiques (hydroxyéthylcellulose, hydroxypropylcellulose, carboxyméthylcellulose..), la gomme de guar et ses dérivés( hydroxypropylguar..), les gommes d'origine microbienne (gomme de xanthane, gomme de scléroglucane..), les épaississants synthétiques tels que les homopolymères réticulés d'acide acrylique ou d'acide acrylamidopropanesulfonique.

Des compositions préférées selon la présente invention comprennent en outre au moins un polymère associatif ionique ou non ionique choisi par exemple parmi les polymères commercialisés sous les appellations PEMULEN ®TR1 ou TR2 par la société GOODRICH, SALCARE SC90® par la société ALLIED COLLOIDS, ACULYN® 22, 28, 33, 44, ou 46 par la société ROHM & HAAS et ELFACOS® T210 et T212 par la société AKZO, dans la proportion de 0,01 à 10% en poids du poids total de la composition.

D'autres compositions préférées selon la présente invention comprennent en outre au moins un polymère cationique ou amphotère bien connu de la technique dans le domaine de la teinture des fibres kératiniques humaines dans la proportion de 0,01 à 10% en poids par rapport au poids total de la composition, de préférence de 0,05 à 5% et plus préférentiellement encore de 0,1 à 3%.

Polymères cationiques

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :

(1) Les homopolymères ou copolymères dérivés d'esters ou d'amides acryliques ou méthacryliques et comportant au moins un des motifs de formules (I), (II), (III) ou (IV) suivantes:

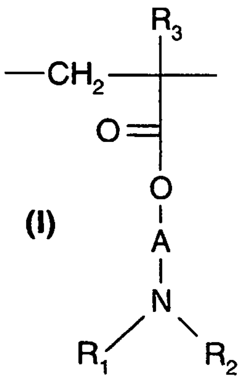
(I)

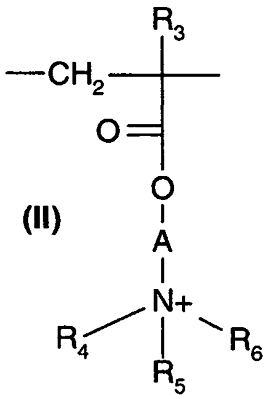
(II)

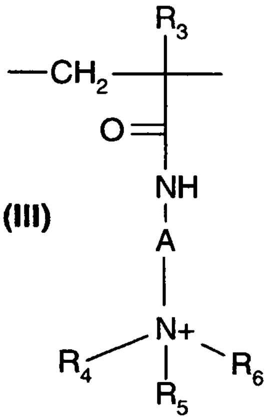
(III)

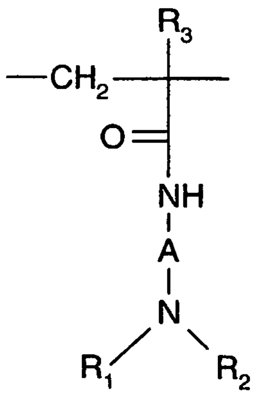
(IV)

dans lesquelles:

$R_3$ , identiques ou différents, désignent un atome d'hydrogène ou un radical $CH_3$;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone;
$R_1$ et $R_2$ , identiques ou différents, représentent hydrogène ou un groupe alkyle ayant de 1 à 6 atomes de carbone et de préférence méthyle ou éthyle;
X désigne un anion dérivé d'un acide minéral ou organique tel qu'un anion méthosulfate ou un halogénure tel que chlorure ou bromure.
Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs ($C_1$-$C_4$), des acides acryliques ou méthacryliques ou leurs esters, des vinyllactames tels que la vinylpyrrolidone ou le vinylcaprolactame, des esters vinyliques.
Ainsi, parmi ces polymères de la famille (1), on peut citer :

- les copolymères d'acrylamide et de diméthylaminoéthyl méthacrylate quaternisé au sulfate de diméthyle ou avec un halogénure de diméthyle, tel que celui vendu sous la dénomination HERCOFLOC par la société HERCULES,
- les copolymères d'acrylamide et de chlorure de méthacryloyloxyéthyltriméthylammonium décrits par exemple dans la demande de brevet EP-A-080976 et vendus sous la dénomination BINA QUAT P 100 par la

société CIBA GEIGY,

- le copolymère d'acrylamide et de méthosulfate de méthacryloyloxyéthyltriméthylammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/ vinylpyrrolidone tel que le produit vendu sous la dénomination GAFFIX VC 713 par la société ISP,
- les copolymère vinylpyrrolidone / méthacrylamidopropyl dimethylamine commercialisés notamment sous la dénomination STYLEZE CC 10 par ISP,
- et les copolymères vinylpyrrolidone / méthacrylamide de diméthylaminopropyle quaternisés tel que le produit vendu sous la dénomination "GAFQUAT HS 100" par la société ISP.

(2) Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .

(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
Les produits commercialisés répondant à cette définition sont plus particulièrement les produits vendus sous la dénomination "Celquat L 200" et "Celquat H 100" par la Société National Starch.

(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
De tels produits sont commercialisés notamment sous les dénominations commerciales de JAGUAR C13 S, JAGUAR C 15, JAGUAR C 17 ou JAGUAR C162 par la société MEYHALL.

(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.

(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .

(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

(8) Les polymères obtenus par réaction d'une polyalkylène polyamine comportant deux groupements amine primaire

et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.

(9) Les cyclopolymères d'alkyl diallyl amine ou de dialkyl diallyl ammonium tels que les homopolymères ou copolymères comportant comme constituant principal de la chaîne des motifs répondant aux formules (V) ou (VI) :

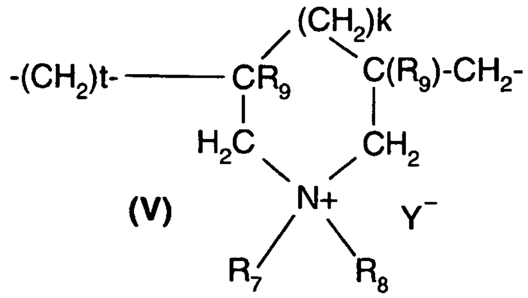

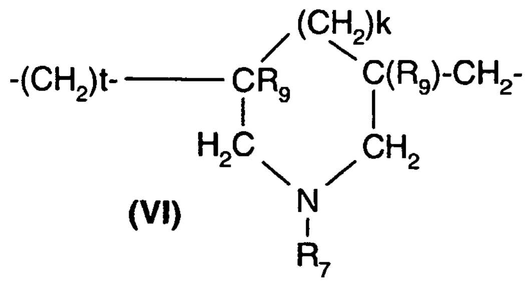

formules dans lesquelles k et t sont égaux à 0 ou 1, la somme k + t étant égale à 1 ; $R_9$ désigne un atome d'hydrogène ou un radical méthyle ; $R_7$ et $R_8$, indépendamment l'un de l'autre, désignent un groupement alkyle ayant de 1 à 8 atomes de carbone, un groupement hydroxyalkyle dans lequel le groupement alkyle a de préférence 1 à 5 atomes de carbone, un groupement amidoalkyle inférieur ($C_1$-$C_4$), ou $R_7$ et $R_8$ peuvent désigner conjointement avec l'atome d'azote auquel ils sont rattachés, des groupement hétérocycliques, tels que pipéridinyle ou morpholinyle ; $R_7$ et $R_8$ indépendamment l'un de l'autre désignent de préférence un groupement alkyle ayant de 1 à 4 atomes de carbone ; $Y^-$ est un anion tel que bromure, chlorure, acétate, borate, citrate, tartrate, bisulfate, bisulfite, sulfate, phosphate.
Ces polymères sont notamment décrits dans le brevet français 2.080.759 et dans son certificat d'addition 2.190.406.
Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".

(10) Le polymère de diammonium quaternaire contenant des motifs récurrents répondant à la formule :

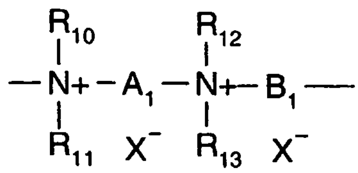

(VII)

formule (VII) dans laquelle :

$R_{10}$, $R_{11}$, $R_{12}$ et $R_{13}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{10}$, $R_{11}$, $R_{12}$ et $R_{13}$, ensemble ou séparément, constituent avec les atomes d'azote auxquels ils sont rattachés des hétérocycles contenant éventuellement un second hétéroatome autre que l'azote ou bien $R_{10}$, $R_{11}$, $R_{12}$ et $R_{13}$ représentent un radical alkyle en $C_1$-$C_6$ linéaire ou ramifié substitué par un groupement nitrile, ester, acyle, amide ou -CO-O- $R_{14}$-D ou -CO-NH-$R_{14}$-D où $R_{14}$ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et $X^-$ désigne un anion dérivé d'un acide minéral ou organique;
A1, $R_{10}$ et $R_{12}$ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement - $(CH_2)n$-CO-D-OC-$(CH_2)n$- dans lequel n est compris entre 1 et 100 et de préférence entre 1 et 50, et D désigne :

a) un reste de glycol de formule : -O-Z-O-, où Z désigne un radical hydrocarboné linéaire ou ramifié ou un groupement répondant à l'une des formules suivantes :

$$-(CH_2-CH_2-O)x-CH_2-CH_2-$$

$$-[CH_2-CH(CH_3)-O]y-CH_2-CH(CH_3)-$$

où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

$$-CH_2-CH_2-S-S-CH_2-CH_2-;$$

d) un groupement uréylène de formule : -NH-CO-NH-.

De préférence, $X^-$ est un anion tel que le chlorure ou le bromure.
Ces polymères ont une masse moléculaire moyenne en nombre généralement comprise entre 1000 et 100000.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
On peut utiliser plus particulièrement les polymères qui sont constitués de motifs récurrents répondant à la formule (VIII) suivante:

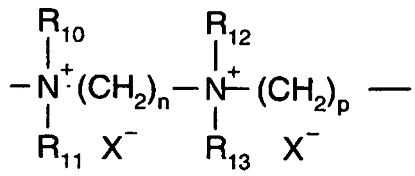

**(VIII)**

dans laquelle $R_{10}$, $R_{11}$, $R_{12}$ et $R_{13,}$ identiques ou différents, désignent un radical alkyle ou hydroxyalkyle ayant de 1 à 4 atomes de carbone environ, n et p sont des nombres entiers variant de 2 à 20 environ et, $X^-$ est un anion dérivé d'un acide minéral ou organique.

(11) Les polyammoniums quaternaires constitués de motifs récurrents de formule (IX):

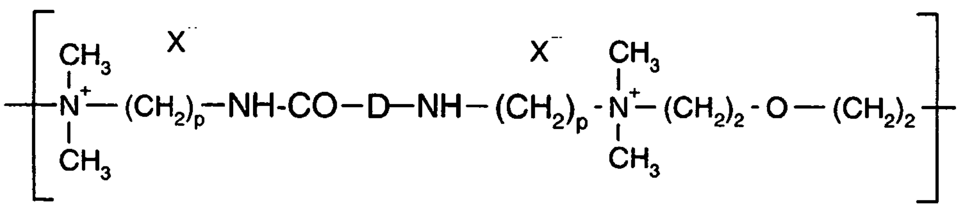

**(IX)**

dans laquelle p désigne un nombre entier variant de 1 à 6 environ, D peut être nul ou peut représenter un groupement $-(CH_2)_r$ -CO- dans lequel r désigne un nombre égal à 4
ou à 7, $X^-$ est un anion ;
De tels polymères peuvent être préparés selon les procédés décrits dans les brevets U.S.A. n° 4 157 388, 4 702 906, 4 719 282. Ils sont notamment décrits dans la demande de brevet EP-A-122 324.
Parmi eux, on peut par exemple citer, les produits "Mirapol A 15", "Mirapol AD1", "Mirapol AZ1" et "Mirapol 175" vendus par la société Miranol.

(12) Les polymères quaternaires de vinylpyrrolidone et de vinylimidazole tels que par exemple les produits commercialisés sous les dénominations Luviquat FC 905, FC 550 et FC 370 par la société B.A.S.F.

(13) Les polyamines comme le Polyquart H vendu par HENKEL, référencé sous le nom de " POLYETHYLENEGLYCOL (15) TALLOW POLYAMINE " dans le dictionnaire CTFA.

(14) Les polymères réticulés de sels de méthacryloyloxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92" par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.
Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes :

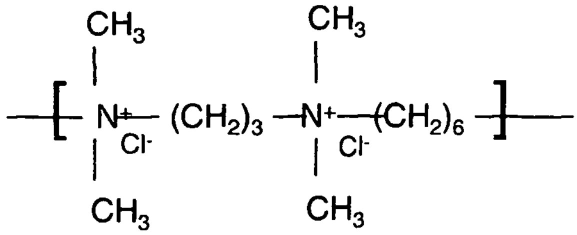

(W)

et notamment ceux dont le poids moléculaire, déterminé par chromatographie par perméation de gel, est compris entre 9500 et 9900;

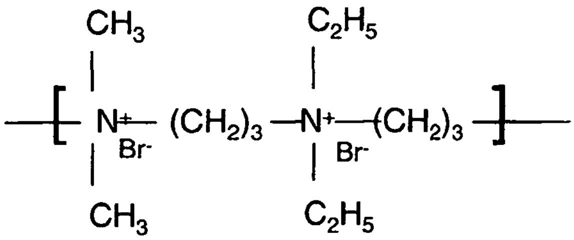

(U)

et notamment ceux dont le poids moléculaire, déterminé par chromatographie par perméation de gel, est d'environ 1200.

La concentration en polymère cationique dans la composition selon la présente invention peut varier de 0,01 à 10% en poids par rapport au poids total de la composition, de préférence de 0,05 à 5% et plus préférentiellement encore de 0,1 à 3%.

Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;

K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène $\alpha,\beta$-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :

(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
Le composé vinylique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.

(2) Les polymères comportant des motifs dérivant :

a) d'au moins un monomère choisi parmi les acrylamides ou les méthacrylamides substitués sur l'azote par un radical alkyle,

b) d'au moins un comonomère acide contenant un ou plusieurs groupements carboxyliques réactifs, et

c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.

Les comonomères basiques préférés sont des méthacrylates d'aminoéthyle, de butyl aminoéthyle, de N,N'-diméthylaminoéthyle, de N-tertio-butylaminoéthyle.

On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.

(3) Les polyaminoamides réticulés et alcoylés partiellement ou totalement dérivant de polyaminoamides de formule générale:

$$-\![CO-R_{19}-CO-Z]\!- \qquad \textbf{(X)}$$

dans laquelle $R_{19}$ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :

a) dans les proportions de 60 à 100 moles %, le radical

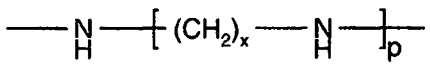

**(XI)**

où x=2 et p=2 ou 3, ou bien x=3 et p=2

ce radical dérivant de la diéthylène triamine, de la triéthylène tétraamine ou de la dipropylène triamine;

b) dans les proportions de 0 à 40 moles % le radical (XI) ci-dessus, dans lequel x=2 et p=1 et qui dérive de l'éthylènediamine, ou le radical dérivant de la pipérazine :

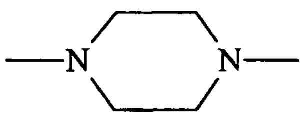

c) dans les proportions de 0 à 20 moles % le radical $-NH-(CH_2)_6-NH-$ dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.

Les alcanes sultones utilisées dans l'alcoylation sont de préférence la propane ou la butane sultone, les sels

des agents d'alcoylation sont de préférence les sels de sodium ou de potassium.

(4) Les polymères comportant des motifs zwittérioniques de formule :

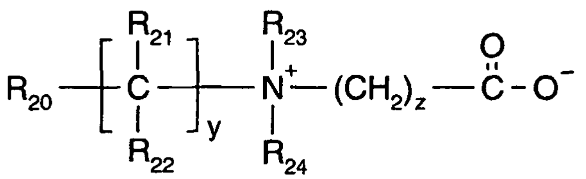

**(XII)**

dans laquelle $R_{20}$ désigne un groupement insaturé polymérisable tel qu'un groupement acrylate, méthacrylate, acrylamide ou méthacrylamide, y et z représentent un nombre entier de 1 à 3, $R_{21}$ et $R_{22}$ représentent un atome d'hydrogène, méthyle, éthyle ou propyle, $R_{23}$ et $R_{24}$ représentent un atome d'hydrogène ou un radical alkyle de telle façon que la somme des atomes de carbone dans $R_{23}$ et $R_{24}$ ne dépasse pas 10.
Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.

(5) les polymères dérivés du chitosane comportant des motifs monomères répondant aux formules (XIII), (XIV), (XV) suivantes :

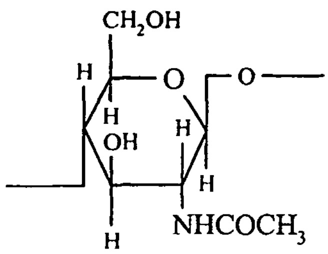

**(XIII)**

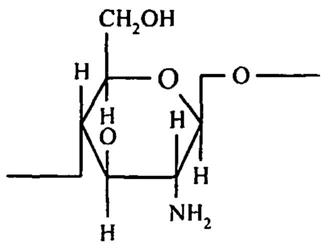

**(XIV)**

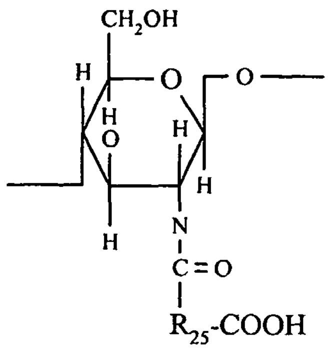

**(XV)**

le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), $R_{25}$ représente un radical de formule :

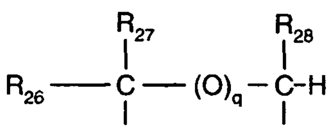

dans laquelle q= 0 ou 1,
si q=0, $R_{26}$, $R_{27}$ et $R_{28}$, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux $R_{26}$, $R_{27}$ et $R_{28}$ étant dans ce cas un atome d'hydrogène ;

ou si q=1, $R_{26}$, $R_{27}$ et $R_{28}$ représentent chacun un atome d'hydrogène, ainsi que les sels formés par ces composés avec des bases ou des acides.

(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.

(7) Les polymères répondant à la formule générale (XI) tels que ceux décrits par exemple dans le brevet français 1 400 366 :

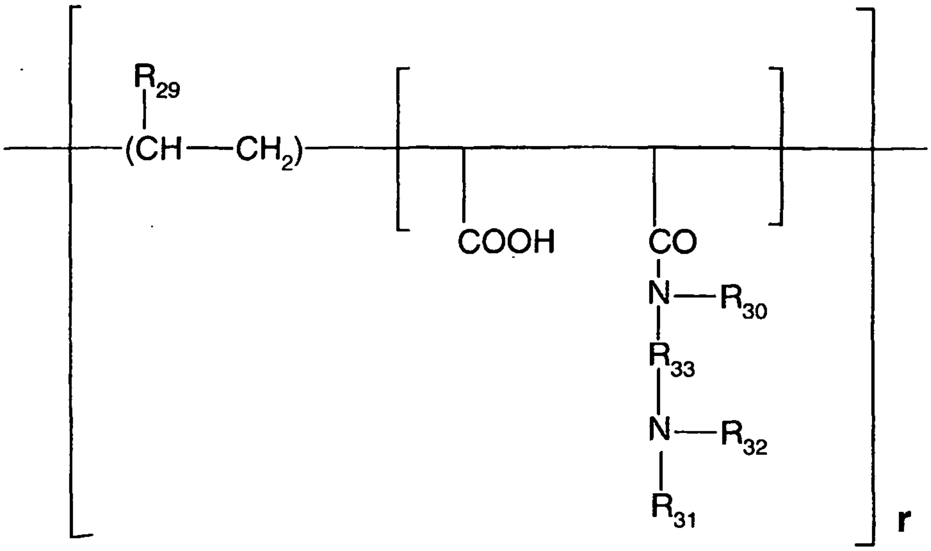

(XVI)

dans laquelle $R_{29}$ représente un atome d'hydrogène, un radical $CH_3O$, $CH_3CH_2O$, phényle, $R_{30}$ désigne l'hydrogène ou un radical alkyle inférieur tel que méthyle, éthyle, $R_{31}$désigne l'hydrogène ou un radical alkyle inférieur tel que méthyle, éthyle, $R_{32}$ désigne un radical alkyle inférieur tel que méthyle, éthyle ou un radical répondant à la formule : $-R_{33}-N(R_{31})_2$, $R_{33}$ représentant un groupement $-CH_2-CH_2-$ , $-CH_2-CH_2-CH_2-$ , $-CH_2-CH(CH_3)-$ , $R_{31}$ avant les significations mentionnées ci-dessus,
ainsi que les homologues supérieurs de ces radicaux et contenant jusqu'à 6 atomes de carbone,
r est tel que le poids moléculaire est compris entre 500 et 6000000 et de préférence entre 1000 et 1000000.

(8) Des polymères amphotères du type -D-X-D-X- choisis parmi:

a) les polymères obtenus par action de l'acide chloracétique ou le chloracétate de sodium sur les composés comportant au moins un motif de formule :

-D-X-D-X-D- (XVII)

où D désigne un radical

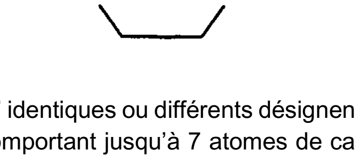

et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool,

ester et/ou uréthanne ;

b) les polymères de formule :

-D-X-D-X- (XVIII)

où D désigne un radical

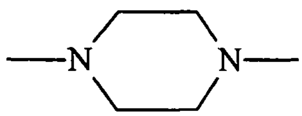

et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) Les copolymères alkyl($C_1$-$C_5$)vinyléther / anhydride maléique modifié partiellement par semiamidification avec une N,N-dialkylaminoalkylamine telle que la N,N-diméthylaminopropylamine ou par semiestérification avec une N, N-dialcanolamine. Ces copolymères peuvent également comporter d'autres comonomères vinyliques tels que le vinylcaprolactame.

Les polymères amphotères particulièrement préférés selon l'invention sont ceux de la famille (1).
Selon l'invention, le ou les polymères amphotères peuvent représenter de 0,01 % à 10 % en poids, de préférence de 0,05 % à 5 % en poids, et encore plus préférentiellement de 0,1 % à 3 % en poids, du poids total de la composition.
Les compositions de l'invention comprennent de préférence un ou plusieurs tensioactifs. Le ou les tensioactifs peuvent être indifféremment choisis, seuls ou en mélanges, au sein des tensioactifs anioniques, amphotères, non ioniques, zwittérioniques et cationiques.
Les tensioactifs convenant à la mise en oeuvre de la présente invention sont notamment les suivants :

(i) <u>Tensioactif(s) anionique(s)</u> :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates ; les alkyl($C_6$-$C_{24}$) sulfosuccinates, les alkyl($C_6$-$C_{24}$) éthersulfosuccinates, les alkyl($C_6$-$C_{24}$) amidesulfosuccinates ; les alkyl($C_6$-$C_{24}$) sulfoacétates ; les acyl($C_6$-$C_{24}$) sarcosinates et les acyl($C_6$-$C_{24}$) glutamates. On peut également utiliser les esters d'alkyl ($C_6$-$C_{24}$)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl ($C_6$-$C_{24}$) éther carboxyliques polyoxyalkylénés, les acides alkyl($C_6$-$C_{24}$)aryl éther carboxyliques polyoxyalkylénés, les acides alkyl($C_6$-$C_{24}$) amido éther carboxyliques polyoxyalkylénés et leurs sels, en particulier ceux comportant de 2 à 50 groupements oxyde d'alkylène en particulier d'éthylène, et leurs mélanges.

(ii) <u>Tensioactif(s) non ionique(s)</u> :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178)

et leur nature ne revet pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine.

(iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

$$R_2\text{-CONHCH}_2\text{CH}_2\text{-N}(R_3)(R_4)(\text{CH}_2\text{COO}^-)$$

dans laquelle : $R_2$ désigne un radical alkyle d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ; et

$$R_2'\text{-CONHCH}_2\text{CH}_2\text{-N(B)(C)}$$

dans laquelle :

B représente $-CH_2CH_2OX'$, C représente $-(CH_2)_z$ -Y', avec z = 1 ou 2,
X' désigne le groupement $-CH_2CH_2-COOH$ ou un atome d'hydrogène
Y' désigne -COOH ou le radical $-CH_2$ - CHOH - $SO_3H$
$R_2'$ désigne un radical alkyle d'un acide Rg -COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL® C2M concentré par la société RHODIA CHIMIE.

(iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent varier de 0,01 à 40% et de préférence de 0,5 à 30% du poids total de la composition.

Bien entendu, l'homme de l'art veillera à choisir le ou les éventuels composés complémentaires mentionnés ci-avant, de manière telle que les propriétés avantageuses attachées intrinsèquement à la composition oxydante selon

l'invention ne soient pas, ou substantiellement pas, altérées par la ou les adjonctions envisagées.

Un autre objet de l'invention est un procédé de teinture d'oxydation des fibres kératiniques humaines et plus particulièrement des cheveux mettant en oeuvre une composition colorante comprenant dans un milieu cosmétiquement acceptable au moins un colorant d'oxydation et une composition oxydante telle que définie ci-dessus.

Selon ce procédé, on applique sur les fibres au moins une composition colorante telle que définie précédemment, la couleur étant révélée à pH acide, neutre ou alcalin à l'aide d'une composition oxydante selon l'invention qui est appliquée simultanément ou séquentiellement, avec ou sans rinçage intermédiaire.

Selon une forme de mise en oeuvre particulièrement préférée du procédé de teinture selon l'invention, on mélange, au moment de l'emploi, la composition colorante décrite ci-dessus avec une composition oxydante selon l'invention. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

Un autre objet de la présente invention est un pr cédé de déformation permanente des fibres kératiniques humaines et plus particulierement des cheveux, utilisant comme composition oxydante la composition définie ci-dessus.

La première étape de ce procédé consiste à appliquer sur les cheveux une composition réductrice. Cette application se fait mèche par mèche ou globalement.

La composition réductrice comprend au moins un agent réducteur, qui peut être en particulier choisi parmi l'acide thioglycolique, la cystéine, la cystéamine, le thioglycolate de glycérol, l'acide thiolactique, ou les sels des acides thiolactique ou thioglycolique.

L'habituelle étape de mise sous tension des cheveux sous une forme correspondant à la forme finale désirée pour ces derniers (boucles par exemple) peut être mise en oeuvre par tout moyen, mécanique notamment, approprié et connu en soi pour maintenir sous tension des cheveux, tels que par exemple rouleaux, bigoudis et analogues.

Les cheveux peuvent également être mis en forme sans l'aide de moyens extérieurs, simplement avec les doigts.

Avant de procéder à l'étape suivante facultative de rinçage, il convient, de manière classique, de laisser reposer pendant quelques minutes, généralement entre 5 minutes et une heure, de préférence entre 10 et 30 minutes, la chevelure sur laquelle a été appliquée la composition réductrice, et ceci de façon à bien laisser le temps au réducteur d'agir correctement sur les cheveux. Cette phase d'attente est effectuée de préférence à une température allant de 35 °C à 45 °C, en protégeant de préférence également les cheveux par un bonnet.

Dans la deuxième étape, facultative, du procédé (étape (ii)), les cheveux imprégnés de la composition réductrice sont donc ensuite rincés soigneusement par une composition aqueuse.

Puis, dans une troisième étape (étape (iii)), on applique sur les cheveux ainsi rincés la composition oxydante selon la présente invention, dans le but de fixer la nouvelle forme imposée aux cheveux.

Comme dans le cas de l'application de la composition réductrice, la chevelure sur laquelle a été appliquée la composition oxydante est ensuite, de manière classique, laissée dans une phase de repos ou d'attente qui dure quelques minutes, généralement entre 3 et 30 minutes, de préférence entre 5 et 15 minutes.

Si la tension des cheveux était maintenue par des moyens extérieurs, on peut retirer de la chevelure ces derniers (rouleaux, bigoudis et analogues) avant ou après l'étape de fixation.

Enfin, dans la dernière étape du procédé selon l'invention (étape (iv)), étape facultative également, les cheveux imprégnés de la composition oxydante sont rincés soigneusement, généralement à l'eau.

On obtient finalement une chevelure facile à démêler, douce. Les cheveux sont ondulés.

La composition oxydante selon l'invention peut également être utilisée dans un procédé de décoloration des fibres kératiniques humaines et plus particulierement des cheveux.

Le procédé de décoloration selon l'invention comprend une étape d'application sur les fibres kératiniques d'une composition oxydante selon l'invention, cette composition comprenant de préférence de l'eau oxygénée en milieu alcalin après mélange extemporané. Classiquement, une deuxième étape du procédé de décoloration selon l'invention est une étape de rinçage des fibres kératiniques.

Les exemples suivants illustrent l'invention sans présenter un caractère limitatif.

**<u>EXEMPLE 1 :</u>**

On a préparé la composition de décoloration aqueuse prête à l'emploi suivante (quantités exprimées en grammes de matière active):

| | |
|---|---|
| Peroxyde d'hydrogène à 200 volumes..... | 12 |
| Stabilisant..... | qs |

(suite)

| | |
|---|---|
| Polydiméthylsiloxane de formule (I) selon l'invention proposé sous la dénomination BELSIL ADM 652® par la société WACKER..... | 2 |
| Agent de pH .....qs..... | pH 4,7 |
| Eau.....qsp..... | 100 |

La composition de décoloration ci-dessus a été appliquée et laissée 45 minutes sous casque sur des cheveux naturels, puis rincées abondamment à l'eau. On a obtenu un éclaircissement régulier de la chevelure et des cheveux dans un état cosmétique très satisfaisant, doux, légers et faciles à démêler.

**EXEMPLE 2 :**

On a préparé la composition de décoloration suivante
( quantités exprimées en grammes de matière active):

**Composition anhydre**

| | |
|---|---|
| Persulfate de potassium..... | 35 |
| Persulfate de sodium..... | 30 |
| Métasilicate de sodium..... | 14 |
| Chlorure d'ammonium..... EDTA..... | 5 |
| Dioctylsulfosuccinate de sodium/benzoate de sodium..... | 1 |
| Stéarate de calcium..... | 1 |
| Polydiméthylsiloxane de formule (II) selon l'invention proposé sous la dénomination FLUID WR 1300® par la société WACKER..... | 2 |
| Silice..... | 7 |

40 g de la composition anhydre ci-dessus ont été mélangés avec 80 g de la composition aqueuse suivante ( quantités exprimées en grammes de matière active):

**Composition aqueuse**

| | |
|---|---|
| Alcool cétéarylique/ceteareth 30.... | 2,85 |
| Stabilisants..... | 0,06 |
| Séquestrant..... | 0,15 |
| Peroxyde d'hydrogène à 200 volumes..... | 9 |
| Acide phosphorique.....qs..... | pH2 |
| Eau distillée.....qsp..... | 100 |

On a obtenu alors une crème décolorante prête à l'emploi qui, appliquée et laissée minutes sous casque, a permis d'obtenir une décoloration homogène de cheveux naturels foncés et dans un état cosmétique très satisfaisant, doux, légers et faciles démêler

**EXEMPLE 3:**

Composition de déformation permanente:

On a préparé la composition réductrice suivante
(quantités exprimées en grammes de matière active) :

| | |
|---|---|
| Acide thioglycolique..... | 9,2 |
| Arginine..... | 15 |
| Ammoniaque à 20% d'NH3..... | 1,86 |
| Carbonate d'ammonium..... | 4,5 |
| Cocoylamidopropylbétaïne/monolaurate de glycérol (25/5) en solution aqueuse à 30%..... | 1,3 |
| Peptisant..... | 0,8 |
| Alcool isostéarylique (TEGO ALKANOL 66 vendu par la société GOLDSCHMIDT)..... | 12 |
| Agent séquestrant..... | 0,4 |
| Parfum..... | 0,4 |
| Eau déminéralisée.....q.s.p..... | 100 |

Cette composition réductrice a été appliquée sur une mèche de cheveux humides, préalablement enroulée sur un bigoudi de 9 mm de diamètre.
Après 10 minutes de temps de pause, on l'a rincée abondamment à l'eau.
On a ensuite appliqué la composition oxydante suivante :

Composition oxydante:

(quantités exprimées en grammes de matière active) :

- Polydiméthylsiloxane de formule (II) selon l'invention proposé sous la dénomination SLM 28020® par la société WACKER 2
- Eau oxygénée 8 volumes
- Pyrophosphate tétrasodique (0,02g ) et stannate de sodium (0,04 g)
- Agent séquestrant : pentaacétate pentasodique 0,06
- Eau déminéralisée.....q.s.p. 100

Après 10 minutes de temps de pause, on a rincé à nouveau abondamment la mèche. Puis on a déroulé les cheveux du bigoudi et on les a séchés.
La mèche a été ondulée avec un très bon état cosmétique.

**Revendications**

1. Composition cosmétique destinée au traitement des fibres kératiniques humaines **caractérisée par le fait qu'**elle comprend dans un milieu cosmétiquement acceptable :

   (i) au moins un agent oxydant et,
   (ii) au moins une silicone aminée de formules (I) ou (II) suivantes :

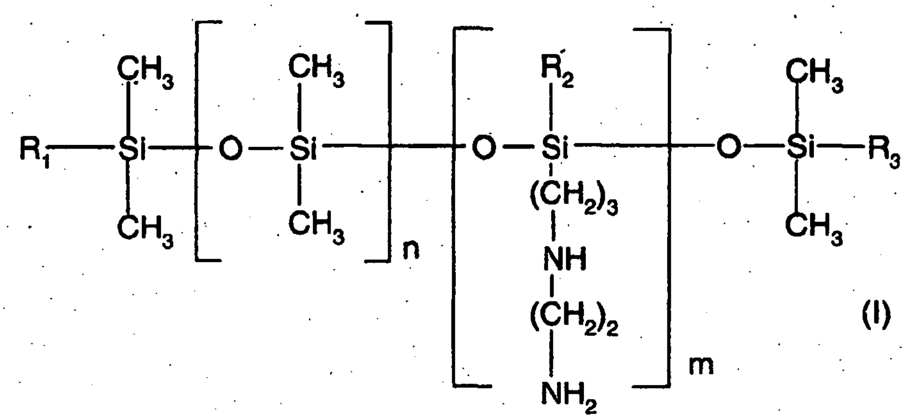

(I)

formule (I) dans laquelle :

m et n sont des nombres tels que la somme (n + m) varie de 1 à 1 000,
n désignant un nombre de 0 à 999 et m désignant un nombre de 1 à 1 000,
$R_1$, $R_2$, $R_3$, identiques ou différents, représentent un radical hydroxy ou alcoxy en $C_1$-$C_4$ ; l'un au moins des radicaux $R_1$ à $R_3$ désignant un radical alcoxy ;

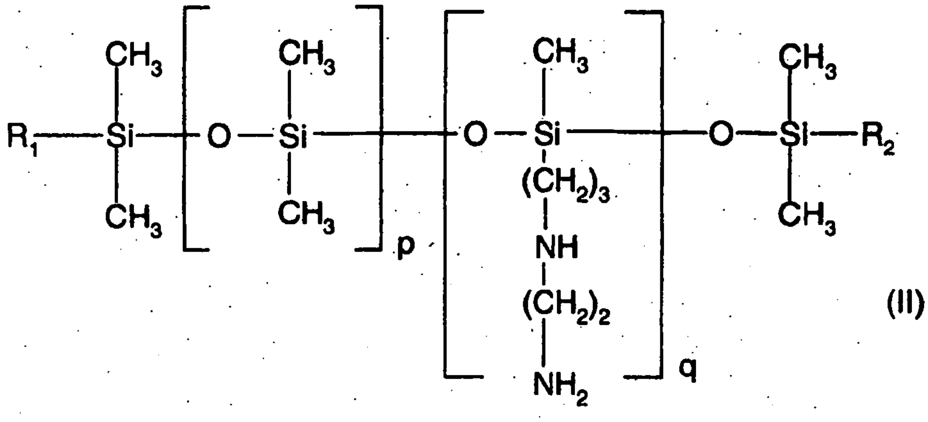

(II)

formule (II) dans laquelle :

p et q sont des nombres tels que la somme (p + q) varie de 1 à 1 000, p désignant un nombre de 0 à 999 et q désignant un nombre de 1 à 1 000 ;
$R_1$, $R_2$, différents, représentent un radical hydroxy ou alcoxy en $C_1$-$C_4$, l'un au moins des radicaux $R_1$ à $R_2$ désignant un radical alcoxy.

**2.** Composition selon la revendication 1, **caractérisée par le fait que** le radical alcoxy $C_1$-$C_4$ désigne le radical méthoxy.

**3.** Composition selon l'une quelconque des revendications précédentes, **caractérisée par** le fait pour les silicones aminées de formule (I), le rapport molaire Hydroxy/Alcoxy est compris entre 0,2:1 et 0,4:1.

**4.** Composition selon l'une quelconque des revendications 1 à 2, **caractérisée par** le fait pour les silicones aminées de formule (II), le rapport molaire Hydroxy/Alcoxy est compris entre 1:0,8 et 1:1,1.

**5.** Composition selon l'une quelconque des revendications 1 à 3, **caractérisée par le fait que** la silicone aminée de formule (I) présente une masse moléculaire moyenne en poids allant de 2000 à 1000000.

**6.** Composition selon l'une quelconque des revendications 1 à 2 et 4, **caractérisée par le fait que** la silicone aminée de formule (II) présente une masse moléculaire moyenne en poids allant de 2000 à 200.000.

**7.** Composition selon l'une quelconque des revendications précédentes, **caractérisée par le fait que** la silicone aminée de formule (I) ou (II) est sous forme d'émulsion huile-dans-eau comprenant des agents tensioactifs.

**8.** Composition selon la revendication 7, **caractérisée par le fait que** l'émulsion comprend au moins un agent tensioactif cationique et/ou non ionique.

**9.** Composition selon l'une quelconque des revendications 7 ou 8, **caractérisée par le fait que** les particules de silicone dans l'émulsion ont une taille allant de 3 nm à 500 nanomètres.

**10.** Composition selon l'une quelconque des revendications précédentes, **caractérisée par le fait que** la silicone aminée de formule (I) ou (II) est présente dans la composition de teinture en une quantité allant de 0,01 à 20% en poids du poids total de la composition.

**11.** Composition selon la revendication 10, **caractérisée par le fait qu'**elle est présente en une quantité allant de 0,1 à 15% en poids en poids du poids total de la composition.

**12.** Composition selon la revendication 11, **caractérisée par le fait qu'**elle est présente en une quantité allant de 0,5 à 10 % en poids du poids total de la composition.

**13.** Composition selon l'une quelconque des revendications précédentes, **caractérisée par le fait que** l'agent oxydant est choisi dans le groupe formé par le peroxyde d'hydrogène, le peroxyde d'urée, les perborates, les persulfates, ou leurs mélanges.

**14.** Composition selon la revendication 13, **caractérisée par le fait que** l'agent oxydant est le peroxyde d'hydrogène.

**15.** Composition selon la revendication 14, **caractérisée par le fait que** la concentration en peroxyde d'hydrogène varie de 0,5 à 40 volumes.

**16.** Composition selon la revendication 15 **caractérisée par le fait que** l'agent oxydant est l'eau oxygénée.

**17.** Composition selon la revendication 16, **caractérisée par le fait que** l'eau oxygénée contient un agent stabilisant.

**18.** Composition selon la revendication 17, **caractérisée par le fait que** l'agent stabilisant est choisi dans le groupe formé par les pyrophosphates de métaux alcalins ou alcalino-terreux, les stannates des métaux alcalins ou alcalino-terreux, la phénacétine, ou les sels d'acides et d'oxyquinoléine.

**19.** Composition selon la revendication 17, **caractérisée par le fait que** l'agent stabilisant est un stannate ou un stannate associé à un pyrophosphate.

**20.** Composition selon l'une quelconque des revendications 17 à 19, **caractérisée par le fait que** la concentration en agent stabilisant varie de 0,0001 à 5% en poids par rapport au poids total de la composition.

**21.** Composition selon l'une quelconque des revendications 17 à 20, **caractérisée par le fait que** le rapport des concentrations du peroxyde d'hydrogène aux agents stabilisants varie de 0,05 à 1000.

**22.** Composition selon l'une quelconque des revendications 17 à 21, **caractérisée par le fait que** le rapport des concentrations de la ou des silicones aminées définies selon les revendications 1 à 12, aux agents stabilisants, varie de 0,05 à 1000.

**23.** Composition selon l'une quelconque des revendications précédentes, **caractérisée par le fait que** le rapport des concentrations de la ou des silicones aminées définies selon les revendications 1 à 12, aux agents oxydants, varie de 0,001 à 10.

**24.** Composition selon l'une quelconque des revendications précédentes, **caractérisée par le fait que** la concentration en oxydant varie de 0,1 à 25% en poids par rapport au poids total de la composition.

**25.** Composition selon l'une quelconque des revendications précédentes, **caractérisée par le fait qu'**elle est aqueuse et que le pH de l'ensemble de la composition varie de 1 à 13.

**26.** Procédé de teinture d'oxydation des fibres kératiniques humaines mettant en oeuvre une composition colorante comprenant dans un milieu cosmétiquement acceptable au moins un colorant d'oxydation et une composition oxydante telle que définie dans l'une quelconque des revendications 1 à 25.

**27.** Procédé de teinture selon la revendication 26 selon lequel on mélange, au moment de l'emploi, la composition colorante avec la composition oxydante ; le mélange obtenu est ensuite appliqué sur les fibres kératiniques et on laisse poser pendant 3 à 50 minutes environ, après quoi on rince, on lave au shampooing, on rince à nouveau et on sèche.

**28.** Procédé de teinture selon la revendication 30 selon lequel on applique séquentiellement la composition colorante et la composition oxydante, avec ou sans rinçage intermédiaire.

**29.** Procédé de traitement des fibres kératiniques humaines en vue d'obtenir une déformation permanente de ces dernières, en particulier sous la forme de cheveux permanentés, ce procédé comprenant les étapes suivantes : (i) on applique sur la matière kératinique à traiter une composition réductrice, la fibre kératinique étant mise sous tension mécanique avant, pendant, ou après ladite application, (ii) on rince éventuellement la fibre kératinique, (iii) on applique sur la fibre kératinique éventuellement rincée une composition oxydante telle que définie à l'une des revendications 1 à 31, (iv) on rince éventuellement à nouveau la fibre kératinique.

**30.** Procédé de décoloration des fibres kératiniques humaines et plus particulièrement des cheveux, comprenant les étapes suivantes : i) on applique sur la fibre kératinique une composition oxydante selon l'une quelconque des revendications 1 à 31, ii) on rince la fibre kératinique ainsi traitée.

**31.** Utilisation d'au moins une silicone aminée de formules (I) ou (II) et telle que définie à l'une quelconque des revendications 1 à 9, dans une composition cosmétique comprenant au moins un agent oxydant, pour améliorer l'état cosmétique des fibres kératiniques humaines.

## Claims

**1.** Cosmetic composition for treating human keratin fibres, **characterized in that** it comprises, in a cosmetically acceptable medium:

(i) at least one oxidizing agent, and
(ii) at least one aminosilicone of formula (I) or (II) below:

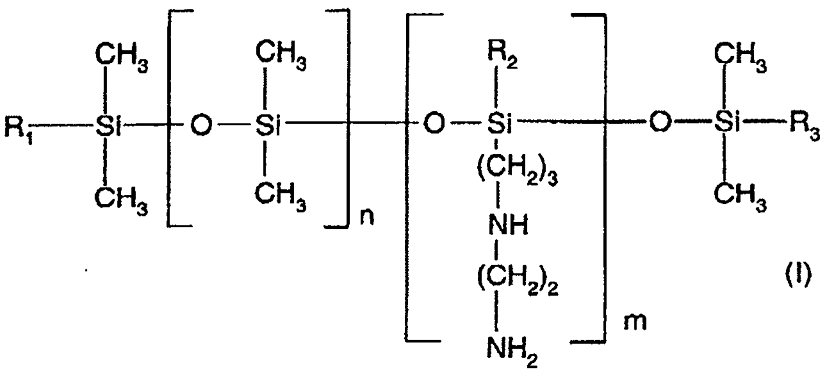

(I)

in which formula (I):

m and n are numbers such that the sum (n + m) ranges from 1 to 1000,
n denoting a number from 0 to 999 and m denoting a number from 1 to 1000;
$R_1$, $R_2$ and $R_3$, which may be identical or different, represent a hydroxyl or $C_1$-$C_4$ alkoxy radical, at least one of the radicals $R_1$ to $R_3$ denoting an alkoxy radical;

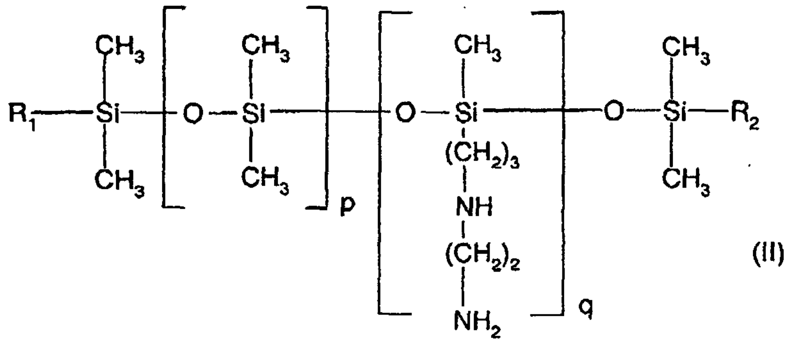

(II)

in which formula (II):

p and q are numbers such that the sum (p + q) ranges from 1 to 1000,
p denoting a number from 0 to 999 and q denoting a number from 1 to 1000;
$R_1$ and $R_2$, which are different, represent a hydroxyl or
$C_1$-$C_4$ alkoxy radical, at least one of the radicals $R_1$ and $R_2$ denoting an alkoxy radical.

2. Composition according to Claim 1, **characterized in that** the $C_1$-$C_4$ alkoxy radical denotes a methoxy radical.

3. Composition according to either of the preceding claims, **characterized in that**, for the aminosilicones of formula (I), the hydroxyl/alkoxy mole ratio is between 0.2:1 and 0.4:1.

4. Composition according to either of Claims 1 and 2, **characterized in that**, for the aminosilicones of formula (II), the hydroxyl/alkoxy mole ratio is between 1:0.8 and 1:1.1.

5. Composition according to any one of Claims 1 to 3, **characterized in that** the aminosilicone of formula (I) has a weight-average molecular mass ranging from 2000 to 1 000 000.

6. Composition according to any one of Claims 1, 2 and 4, **characterized in that** the aminosilicone of formula (II) has a weight-average molecular mass ranging from 2000 to 200 000.

7. Composition according to any one of the preceding claims, **characterized in that** the aminosilicone of formula (I) or (II) is in the form of an oil-in-water emulsion comprising surfactants.

8. Composition according to Claim 7, **characterized in that** the emulsion comprises at least one cationic and/or nonionic surfactant.

9. Composition according to either of Claims 7 and 8, **characterized in that** the silicone particles in the emulsion range in size from 3 nm to 500 nanometres.

10. Composition according to any one of the preceding claims, **characterized in that** the aminosilicone of formula (I) or (II) is present in the dye composition in an amount ranging from 0.01% to 20% by weight relative to the total weight of the composition.

11. Composition according to Claim 10, **characterized in that** it is present in an amount ranging from 0.1% to 15% by weight relative to the total weight of the composition.

12. Composition according to Claim 11, **characterized in that** it is present in an amount ranging from 0.5% to 10% by weight relative to the total weight of the composition.

13. Composition according to any one of the preceding claims, **characterized in that** the oxidizing agent is chosen from the group formed by hydrogen peroxide, urea peroxide, perborates and persulfates, or mixtures thereof.

14. Composition according to Claim 13, **characterized in that** the oxidizing agent is hydrogen peroxide.

**15.** Composition according to Claim 14, **characterized in that** the hydrogen peroxide concentration ranges from 0.5 to 40 volumes.

**16.** Composition according to Claim 15, **characterized in that** the oxidizing agent is aqueous hydrogen peroxide solution.

**17.** Composition according to Claim 16, **characterized in that** the aqueous hydrogen peroxide solution contains a stabilizer.

**18.** Composition according to Claim 17, **characterized in that** the stabilizer is chosen from the group formed by alkali metal or alkaline-earth metal pyrophosphates, alkali metal or alkaline-earth metal stannates, phenacetin, or salts of acids and of oxyquinoline.

**19.** Composition according to Claim 17, **characterized in that** the stabilizer is a stannate or a stannate combined with a pyrophosphate.

**20.** Composition according to any one of Claims 17 to 19, **characterized in that** the stabilizer concentration ranges from 0.0001% to 5% by weight relative to the total weight of the composition.

**21.** Composition according to any one of Claims 17 to 20, **characterized in that** the concentration ratio of hydrogen peroxide to the stabilizers ranges from 0.05 to 1000.

**22.** Composition according to any one of Claims 17 to 21, **characterized in that** the concentration ratio of the aminosilicone(s) defined according to Claims 1 to 12, to the stabilizers, ranges from 0.05 to 1000.

**23.** Composition according to any one of the preceding claims, **characterized in that** the concentration ratio of the aminosilicone(s) defined according to Claims 1 to 12, to the oxidizing agents, ranges from 0.001 to 10.

**24.** Composition according to any one of the preceding claims, **characterized in that** the oxidizing agent concentration ranges from 0.1% to 25% by weight relative to the total weight of the composition.

**25.** Composition according to any one of the preceding claims, **characterized in that** it is aqueous and **in that** the pH of the composition as a whole ranges from 1 to 13.

**26.** Process for the oxidation dyeing of human keratin fibres, using a dye composition comprising, in a cosmetically acceptable medium, at least one oxidation dye and an oxidizing composition as defined in any one of Claims 1 to 25.

**27.** Dyeing process according to Claim 26, according to which the dye composition is mixed, at the time of use, with the oxidizing composition; the mixture obtained is then applied to the keratin fibres and is left to act for 3 to 50 minutes approximately, after which the fibres are rinsed, washed with shampoo, rinsed again and dried.

**28.** Dyeing process according to Claim 27, according to which the dye composition and the oxidizing composition are applied sequentially, with or without intermediate rinsing.

**29.** Process for treating human keratin fibres, in order to permanently reshape the said fibres, in particular in the form of permanent-waved hair, this process comprising the following steps: (i) a reducing composition is applied to the keratin material to be treated, the keratin fibre being placed under mechanical tension before, during or after the said application, (ii) the keratin fibre is optionally rinsed, (iii) an oxidizing composition as defined in one of Claims 1 to 25 is applied to the optionally rinsed keratin fibre, (iv) the keratin fibre is optionally rinsed again.

**30.** Process for bleaching human keratin fibres, and more particularly the hair, comprising the following steps: i) an oxidizing composition according to any one of Claims 1 to 25 is applied to the keratin fibre, ii) the keratin fibre thus treated is rinsed.

**31.** Use of at least one aminosilicone of formula (I) or (II) as defined in any one of Claims 1 to 9, in a cosmetic composition comprising at least one oxidizing agent, to improve the cosmetic condition of human keratin fibres.

## Patentansprüche

1. Kosmetische Zusammensetzung, die für die Behandlung von menschlichen Keratinfasern vorgesehen ist, **dadurch gekennzeichnet, dass** sie in einem kosmetisch akzeptablen Medium enthält:

   (i) mindestens ein Oxidationsmittel und
   (ii) mindestens ein aminiertes Silicon der folgenden Formeln (I) oder (II) :

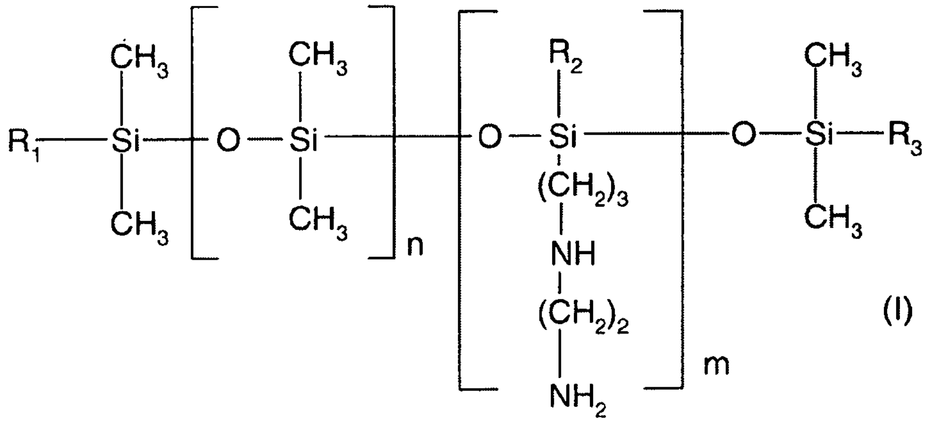

(I)

in der Formel (I):

m und n bedeuten Zahlen, die so gewählt sind, dass die Summe (n + m) im Bereich von 1 bis 1000 liegt, n ist eine Zahl im Bereich von 0 bis 999 und m ist eine Zahl im Bereich von 1 bis 1000,
$R_1$, $R_2$ und $R_3$, die gleich oder verschieden sind, bedeuten eine Hydroxygruppe oder eine $C_{1-4}$-Alkoxygruppe, wobei mindestens eine der Gruppen $R_1$ bis $R_3$ eine Alkoxygruppe bedeutet;

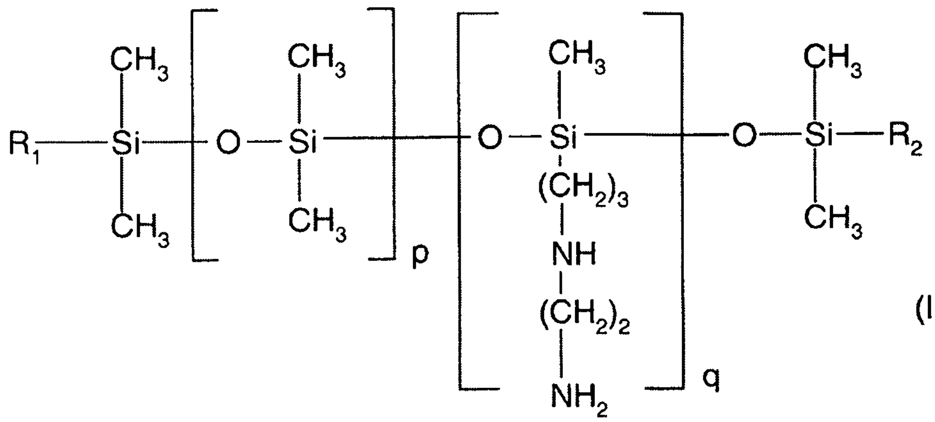

(II)

in der Formel (II):

p und q bedeuten Zahlen, die so gewählt sind, dass die Summe (p + q) im Bereich von 1 bis 1000 liegt, p ist eine Zahl im Bereich von 0 bis 999 und q ist eine Zahl im Bereich von 1 bis 1000,
$R_1$ und $R_2$, die verschieden sind, bedeuten eine Hydroxygruppe oder eine $C_{1-4}$-Alkoxygruppe, wobei mindestens eine der Gruppen $R_1$ bis $R_2$ eine Alkoxygruppe bedeutet.

2. Zusammensetzung nach Anspruch 1, **dadurch gekennzeichnet, dass** die $C_{1-4}$-Alkoxygruppe die Methoxygruppe bedeutet.

3. Zusammensetzung nach einem der vorhergehenden Ansprüche, **dadurch gekennzeichnet, dass** für die aminierten

Silicone der Formel (I) das Molverhältnis Hydroxy/Alkoxy im Bereich von 0,2:1 bis 0,4:1 liegt.

**4.** Zusammensetzung nach einem der Ansprüche 1 bis 2, **dadurch gekennzeichnet, dass** für die aminierten Silicone der Formel (II) das Molverhältnis Hydroxy/Alkoxy im Bereich von 1:0,8 bis 1:1,1 1 liegt.

**5.** Zusammensetzung nach einem der Ansprüche 1 bis 3, **dadurch gekennzeichnet, dass** das aminierte Silicon der Formel (I) eine gewichtmittlere Molmasse von 2.000 bis 1.000.000 aufweist.

**6.** Zusammensetzung nach einem der Ansprüche 1 bis 2 und 4, **dadurch gekennzeichnet, dass** das aminierte Silicon der Formel (II) eine gewichtmittlere Molmasse von 2.000 bis 200.000 aufweist.

**7.** Zusammensetzung nach einem der vorhergehenden Ansprüche, **dadurch gekennzeichnet, dass** das aminierte Silicon der Formel (I) oder (II) in Form einer Öl-in-Wasser-Emulsion vorliegt, die einen oder mehrere grenzflächenaktive Stoffe enthält.

**8.** Zusammensetzung nach Anspruch 7, **dadurch gekennzeichnet, dass** die Emulsion mindestens einen kationischen und/oder nichtionischen grenzflächenaktiven Stoff enthält.

**9.** Zusammensetzung nach einem der Ansprüche 7 oder 8, **dadurch gekennzeichnet, dass** die Siliconpartikel in der Emulsion eine Größe im Bereich von 3 bis 500 nm aufweisen.

**10.** Zusammensetzung nach einem der vorhergehenden Ansprüche, **dadurch gekennzeichnet, dass** das aminierte Silicon der Formel (I) oder (II) in der Farbmittelzusammensetzung in einem Mengenanteil von 0,01 bis 20 Gew.-% des Gesamtgewichts der Zusammensetzung enthalten ist.

**11.** Zusammensetzung nach Anspruch 10, **dadurch gekennzeichnet, dass** es in einem Mengenanteil von 0,1 bis 15 Gew.-% des Gesamtgewichts der Zusammensetzung enthalten ist.

**12.** Zusammensetzung nach Anspruch 11, **dadurch gekennzeichnet, dass** es in einem Mengenanteil von 0,5 bis 10 Gew.-% des Gesamtgewichts der Zusammensetzung enthalten ist.

**13.** Zusammensetzung nach einem der vorhergehenden Ansprüche, **dadurch gekennzeichnet, dass** das Oxidationsmittel unter Wasserstoffperoxid, Harnstoffperoxid, Perboraten, Persulfaten oder deren Gemischen ausgewählt ist.

**14.** Zusammensetzung nach Anspruch 13, **dadurch gekennzeichnet, dass** es sich bei dem Oxidationsmittel um Wasserstoffperoxid handelt.

**15.** Zusammensetzung nach Anspruch 14, **dadurch gekennzeichnet, dass** die Konzentration des Wasserstoffperoxids im Bereich von 0,5 bis 40 Volumina liegt.

**16.** Zusammensetzung nach Anspruch 15, **dadurch gekennzeichnet, dass** es sich bei dem Oxidationsmittel um eine wässrige Wasserstoffperoxidlösung handelt.

**17.** Zusammensetzung nach Anspruch 16, **dadurch gekennzeichnet, dass** die wässrige Wasserstoffperoxidlösung einen Stabilisator enthält.

**18.** Zusammensetzung nach Anspruch 17, **dadurch gekennzeichnet, dass** der Stabilisator unter den Alkalimetall- oder Erdalkalimetallpyrophosphaten, Alkalimetall- oder Erdalkalimetallstannaten, Phenacetin oder Salzen von Säuren und Oxychinolin ausgewählt sind.

**19.** Zusammensetzung nach Anspruch 17, **dadurch gekennzeichnet, dass** der Stabilisator ein Stannat oder ein Stannat im Kombination mit einem Pyrophosphat ist.

**20.** Zusammensetzung nach einem der Ansprüche 17 bis 19, **dadurch gekennzeichnet, dass** die Konzentration des Stabilisators im Bereich von 0,0001 bis 5 Gew.-%, bezogen auf das Gesamtgewicht der Zusammensetzung, liegt.

**21.** Zusammensetzung nach einem der Ansprüche 17 bis 20, **dadurch gekennzeichnet, dass** das Verhältnis der Konzentrationen des Wasserstoffperoxids und der Stabilisatoren im Bereich von 0,05 bis 1000 liegt.

22. Zusammensetzung nach einem der Ansprüche 17 bis 21, **dadurch gekennzeichnet, dass** das Verhältnis der Konzentrationen des oder der in den Ansprüchen 1 bis 12 definierten aminierten Silicone und der Stabilisatoren im Bereich von 0,05 bis 1000 liegt.

23. Zusammensetzung nach einem der vorhergehenden Ansprüche, **dadurch gekennzeichnet, dass** das Verhältnis der Konzentrationen des oder der in den Ansprüchen 1 bis 12 definierten aminierten Silicone und der Oxidationsmittel im Bereich von 0,001 bis 10 liegt.

24. Zusammensetzung nach einem der vorhergehenden Ansprüche, **dadurch gekennzeichnet, dass** die Konzentration des Oxidationsmittels im Bereich von 0,1 bis 25 Gew.-%, bezogen auf das Gesamtgewicht der Zusammensetzung, liegt.

25. Zusammensetzung nach einem der vorhergehenden Ansprüche, **dadurch gekennzeichnet, dass** sie wässrig ist und **dadurch**, dass der pH-Wert der gesamten Zusammensetzung im Bereich von 1 bis 13 liegt.

26. Verfahren zum oxidativen Färben menschlicher Keratinfasern, bei dem eine Farbmittelzusammensetzung, die in einem kosmetisch akzeptablen Medium mindestens einen Oxidationsfarbstoff enthält, und eine oxidierende Zusammensetzung verwendet wird, wie sie in einem der Ansprüche 1 bis 25 definiert ist.

27. Verfahren zum Färben nach Anspruch 26, bei dem bei der Anwendung die Farbmittelzusammensetzung mit der oxidierenden Zusammensetzung vermischt wird, das erhaltene Gemisch dann auf die Keratinfasern aufgetragen wird und während einer Zeitspanne von etwa 3 bis 50 min einwirken gelassen wird, worauf gespült, mit Haarwaschmittel gewaschen, nochmals gespült und getrocknet wird.

28. Verfahren zum Färben nach Anspruch 27, bei dem die Farbmittelzusammensetzung und die oxidierende Zusammensetzung ohne zwischenzeitliches Spülen nacheinander aufgetragen werden.

29. Verfahren zur Behandlung menschlicher Keratinfasern, um diese dauerhaft zu verformen, insbesondere in Form von dauergewellten Haaren, wobei dieses Verfahren die folgenden Schritte umfasst: (i) auf die zu behandelnde Keratinsubstanz wird eine reduzierende Zusammensetzung aufgetragen, wobei die Keratinfaser vor, während oder nach dieser Anwendung unter mechanische Spannung gesetzt wird, (ii) die Keratinfaser wird gegebenenfalls gespült, (iii) auf die gegebenenfalls gespülte Keratinfaser wird eine oxidierende Zusammensetzung aufgetragen, wie sie in einem der Ansprüche 1 bis 31 definiert ist, und (iv) die Keratinfaser wird gegebenenfalls nochmals gespült.

30. Verfahren zum Entfärben von menschlichen Keratinfasern und insbesondere Haaren, das die folgenden Schritte umfasst: (i) auf die Keratinfaser wird eine oxidierende Zusammensetzung aufgetragen, wie sie in einem der Ansprüche 1 bis 31 definiert ist, ii) die auf diese Weise behandelte Keratinfaser wird gespült.

31. Verwendung mindestens eines aminierten Silicons der Formeln (I) oder (II), wie es in einem der Ansprüche 1 bis 9 definiert ist, in einer kosmetischen Zusammensetzung, die mindestens ein Oxidationsmittel enthält, um den kosmetischen Zustand menschlicher Keratinfasern zu verbessern.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 890335 A
- EP 337354 A
- FR 2270846
- FR 2383660
- FR 2598611
- FR 2470596
- FR 2519863
- FR 2505348
- FR 2542997
- EP 080976 A
- FR 2077143
- FR 2393573
- FR 1492597
- US 4131576 A
- US 3589578 A
- US 4031307 A
- FR 2162025
- FR 2280361
- FR 2252840
- FR 2368508
- FR 1583363
- US 3227615 A
- US 2961347 A
- FR 2080759
- FR 2320330
- FR 2316271
- FR 2336434
- FR 2413907
- US 2273780 A
- US 2375853 A
- US 2388614 A
- US 2454547 A
- US 3206462 A
- US 2261002 A
- US 2271378 A
- US 3874870 A
- US 4001432 A
- US 3929990 A
- US 3966904 A
- US 4005193 A
- US 4025617 A
- US 4025627 A
- US 4025653 A
- US 4026945 A
- US 4027020 A
- US 4157388 A
- US 4702906 A
- US 4719282 A
- EP 122324 A
- FR 1400366
- US 2528378 A
- US 2781354 A

### Littérature non-brevet citée dans la description

- CTFA. 1991
- **M.R. PORTER.** Handbook of Surfactants. Blackie & Son, 1991, 116-178
- CTFA. 1993